# EUROPÄISCHE PATENTSCHRIFT

(11) **EP 4 213 710 B1**
(45) Veröffentlichungstag und Bekanntmachung des Hinweises auf die Patenterteilung: **03.07.2024**
(21) Anmeldenummer: 21844957.7
(22) Anmeldetag: 16.12.2021
(51) Int. Cl.: A61B 5/00, A61B 5/0205, A61B 5/03

(54) **MESSVORRICHTUNG ZUM NICHT-INVASIVEN ERFASSEN DES SCHÄDELINNENDRUCKS EINES PATIENTEN UND ZUGEHÖRIGES VERFAHREN**
MEASURING DEVICE FOR NON-INVASIVELY DETECTING THE INTRACRANIAL PRESSURE OF A PATIENT, AND CORRESPONDING METHOD
DISPOSITIF DE MESURE POUR LA DÉTECTION NON INVASIVE DE LA PRESSION INTRACRÂNIENNE D'UN PATIENT ET PROCÉDÉ ASSOCIÉ

(30) Priorität: 16.12.2020 DE 102020133776
(43) Veröffentlichungstag der Anmeldung: 26.07.2023
(73) Patentinhaber: iNDTact GmbH, 97076 Würzburg (DE)
(72) Erfinder: PETRICEVIC, Raino, 97074 Würzburg (DE); LAUNER, Clemens, 97076 Würzburg (DE)
(74) Vertreter: Dr. Gassner & Partner mbB
(86) Internationale Anmeldenummer: PCT/EP2021/086314
(87) Internationale Veröffentlichungsnummer: WO 2022/129415

(56) Entgegenhaltungen:
- WO-A1-2018/170132
- CN-A- 104 720 863
- US-A- 4 600 855
- US-A1- 2012 088 957
- US-A1- 2013 085 400
- US-A1- 2017 089 782

## Beschreibung

Die Erfindung betrifft eine Messvorrichtung zum nicht-invasiven Erfassen der Hirninnendruckpulsation eines Patienten.

Zahlreiche neurointensivmedizinische Erkrankungen können mit einer lebensbedrohlichen Erhöhung des intrakraniellen Drucks (intracranial pressure, ICP) einhergehen. Da das Volumen im Schädelinneren konstant ist, kann die Volumenzunahme eines oder mehrerer Kompartimente zu einer ICP-Erhöhung führen. Zu diesen Kompartimenten gehören das Hirngewebe (z. B. durch Blutung, Schwellung, Entzündung), der Liquorraum (z. B. durch Hydrozephalus, Blutung) und das Gefäßkompartiment (z. B. Änderung durch Hyper- oder Hypoventilation). Der Zusammenhang zwischen intrakraniellem Volumen und intrakraniellem Druck wird als intrakranielle Compliance bezeichnet. Der ICP steigt bei Volumenzunahme exponentiell, da zunächst durch sogenannte Reserveräume (Liquorraum, Gefäßkompartiment) ein ICP-Anstieg kompensiert werden kann (Monroe-Kellie-Doktrin). Erkrankungen, die zu einer Druckerhöhung führen können, sind unter anderem das Schädel-Hirn-Trauma, epi- und subdurale Hämatome, raumfordernde ischämische Schlaganfälle, die intrazerebrale Blutung, die Subarachnoidalblutung, Sinus- und Hirnvenenthrombosen, Meningitiden, Enzephalitiden, die globale zerebrale Hypoxie sowie andere Entitäten wie Hirntumoren, Intoxikationen und metabolische Störungen.

Um den ICP in kritischen Fällen wie beispielsweise bei schwerem Schädel-Hirn-Trauma permanent zu überwachen, kann ein Messkatheter invasiv durch die Schädelkalotte eingeführt werden. Invasive Messverfahren stellen für viele Patienten allerdings eine große Belastung dar, so dass auf eine Überwachung oft verzichtet wird.

Es sind bereits nicht-invasive Messverfahren vorgeschlagen worden, die auf der Messung der Dehnung des Schädels beruhen. Blutvolumenschwankungen durch den Herzschlag führen zur Dehnung des Schädels, vor allem über die bindegewebig geschlossenen Schädelnähte. Die dadurch entstehenden Druckpulsschwankungen im Gehirn betragen ca. 3-4 mmHg. Diese bewirken eine minimale pulssynchrone Ausdehnung des Schädels.

Das Dokument WO 2013/041973 A2 schlägt eine Messvorrichtung zur nicht-invasiven Messung des intrakraniellen Drucks vor, die einen Sensor umfasst, der zum Erfassen der Verformung des Schädels ausgebildet ist. Der Sensor ist mit einem Verstärker, einem A/D-Wandler, einem Prozessor, einer Anzeige und einem Speicher verbunden. Die Messvorrichtung ermöglicht die Feststellung des intrakraniellen Drucks durch Auswerten der Sensorsignale, anhand denen die Verformung des Schädels festgestellt werden kann.

Aus der WO 2019/087148 A1 ist eine ähnliche Messvorrichtung bekannt, bei der von einem Sensor erfasste Daten nach einer Verarbeitung drahtlos an einen Empfänger gesendet werden.

Die US 2013/085400 A1 schlägt eine Messvorrichtung zum nicht-invasiven Erfassen der Hirninnendruckpulsation eines Patienten vor. Zur Messung können unterschiedliche Arten von Sensoren verwendet werden, z. B. ein Dehnungssensor.

US 4 600 855 A offenbart eine Vorrichtung vor, mit der der Druck einer Körperflüssigkeit eines Patienten gemessen werden kann. Beispielsweise kann der Blutdruck oder der interkranielle Druck eines Patienten mit der Vorrichtung überwacht werden. Die Messung erfolgt durch piezoelektrische Übertrager.

US 2012/088957 A1 offenbart einen Aktuator für eine subkutane piezoelektrische Hörhilfe mit Knochenleitung. Zur Messung dient ein piezoelektrisches Element, das zum Erfassen von Biegung ausgebildet ist.

WO 2018/170132 A1 offenbart einen biologisch abbaubaren implantierbaren Drucksensor zur Messung von Vitalparametern eines Patienten.

Bei diesen Messvorrichtungen besteht allerdings der Nachteil, dass ein dominanter Einfluss der Pulsation der Arteria Carotis Externa aufgrund einer fehlenden Entkopplung nicht ausgeschlossen werden kann. Die durch den pulsierenden Innendruck verursachte Schädelpulsation, welche deutlich geringer als die arterielle Pulsation ist, fällt ohne Diskriminierung der arteriellen Pulsation kaum ins Gewicht. Die in den zitierten Druckschriften vorgeschlagenen Dehnungsmessanordnungen werden an ihrer Messgrenze betrieben. Damit ist auch keine Polsterung der Messvorrichtung am Schädel möglich, sodass ein längerer Einsatz für den Patienten mit zunehmender Dauer sehr unbequem ist.

Der Erfindung liegt die Aufgabe zugrunde, eine Messvorrichtung zum nicht-invasiven Erfassen der Hirninnendruckpulsation anzugeben, die die genannten Nachteile beseitigt und eine einfache und dennoch zuverlässige Messung von Vitaldaten wie den statischen Hirninnendruck ermöglicht.

Zur Lösung dieser Aufgabe ist eine Messvorrichtung mit den Merkmalen des Anspruchs 1 vorgesehen.

Die erfindungsgemäße Messvorrichtung umfasst eine Haltevorrichtung, die kraft- und/oder formschlüssig an der Außenseite des Schädels des Patienten lösbar anbringbar ist, wenigstens einen zwei Sensorschichten mit antiparalleler Polarität, die symmetrisch um die neutrale Faser angeordnet sind, aufweisenden bimorphen Biegesensor, der in oder an der Haltevorrichtung angeordnet ist, wobei bei Biegung in eine Richtung eine der Biegesensorschichten gedehnt wird, während die andere gleichermaßen gestaucht wird, wobei sich die Signale bei gegenläufigen Belastungen der Biegesensorschichten addieren, einen Analogsignalverstärker zum Verstärken der von dem bimorphen Biegesensor gelieferten Messdaten, - einen A/D-Wandler zum Umwandeln der analogen Messdaten in digitale Daten, und eine Recheneinheit zur Vorverarbeitung der Daten und Berechnung von Parametern aus dem Hirninnendruckpulsationsverlauf, welche mit Vitalzustandsgrößen korrelieren, anhand der digitalen Daten.

Die erfindungsgemäße Messvorrichtung zeichnet sich dadurch aus, dass sie den Einfluss pulsierender arterieller Gefäße weitgehend eliminiert, d. h. es wird tatsächlich die Hirninnendruckpulsation gemessen. Die Recheneinheit dient neben der Konditionierung der digitalen Daten auch zur Durchführung von Korrekturen und zur Berechnung charakteristischer Kurvenparameter und daraus ableitbarer Werte wie zum Beispiel des Schädelinnendrucks (ICP). Daneben können systolische oder diastolische Kennwerte oder Vitalparameter aus den digitalen Daten abgeleitet werden. Optional umfasst die erfindungsgemäße Messvorrichtung eine Anzeige, sodass Amplitudenverläufe, eine Messkurve, ermittelte Parameter oder abgeleitete Werte oder Warnungen ausgegeben werden können.

Die Erfindung beruht auf der Erkenntnis, dass mit einem bimorphen Biegesensor eine besonders einfache und dennoch zuverlässige und präzise Messung der Schädelinnendruckpulsation durchgeführt werden kann. Das Prinzip der Erfindung beruht auf der Auslenkung eines an einer Kopfmanschette oder an einer Auflagefläche am Kopf angebrachten bimorphen Biegesensors aufgrund der pulssynchronen Schädelvolumenausdehnung durch den pulsierenden Schädelinnendruck (ICP).

Ein piezoelektrischer Biegesensor kann kleinste Verformungen bzw. Schwingungen des Schädels aufgrund des Herzschlags messen. Der Druck des durch das Herz in das Gehirn gepumpten Blutes nimmt während des Pulsschlags aufgrund der Übertragungswege durch das Hirngewebe stufenförmig ab. Die Übertragungsfunktion hängt mitunter vom Schädelinnendruck und damit verbundenen Autoregulationsmechanismen ab, so dass aus dem dynamischen Verlauf des Druckabfalls unter anderem auf den Schädelinnendruck und den Autoregulationsstatus geschlossen werden kann.

Vorzugsweise ist die Haltevorrichtung als Stirnband oder Kopfmanschette ausgebildet. Allerdings kann die Haltevorrichtung alternativ auch durch Auflegen, Aufkleben oder Klemmen des Biegesensors mittels einer Mütze oder eines Verbands angebracht werden. Denkbar ist dabei auch die ausschließliche oder zusätzliche Verwendung eines geeigneten elastischen Kopplungsmediums wie ein hautfreundlicher doppelseitiger Klebefilm.

Die Messvorrichtung umfasst das Stirnband oder die Manschette, die zumindest teilweise biegeflexibel ist und auch zumindest teilweise elastisch dehnbar sein kann. Die Spannkraft des Stirnbands oder der Manschette ist einstellbar. Der wenigstens eine Biegesensor ist Teil eines biegeflexiblen Bereichs des Stirnbands oder der Manschette und kann sich durch statische bzw. dynamische Volumenausdehnung des Schädels direkt oder aufgrund einer damit verbundenen Zugspannungsänderung der daran befestigten Halterung, insbesondere eines Stirnbands, statisch oder dynamisch verbiegen. Die Manschette kann an den Kopf des Patienten angelegt und über eine Spannvorrichtung mit konstanter Spannkraft befestigt werden.

Vorzugsweise ist der Biegesensor ein bimorpher piezoelektrischer Biegesensor. Es handelt sich dabei um einen bimorphen Biegesensor mit antiparalleler Polarität. Dabei wird von dem Effekt Gebrauch gemacht, dass die druckpulsbedingte dynamische Volumenauslenkung eines Schädels, an dem die erfindungsgemäße Messvorrichtung angeordnet ist, eine dynamische Zugspannung auf die als Stirnband ausgebildete erfindungsgemäße Messvorrichtung und eine dynamische Biegung an der Position des Biegesensors verursacht. Auf diese Weise können auch extrem schwache druckbedingte Volumenänderungen des Schädels detektiert werden.

Ein bimorpher Biegesensor besteht aus zwei Sensorschichten, die symmetrisch um die neutrale Faser angeordnet sind. Bei Biegung dieser Anordnung in eine Richtung, wird eine der sensorisch aktiven Biegesensorschichten gedehnt, während die andere gleichermaßen gestaucht wird. Bei Biegung in die andere Richtung verhält es sich genau umgekehrt. Durch eine antiparallele Polarität der zwei Sensorschichten addieren sich die Signale dieser gegenläufigen Belastungen, da sie das gleiche Vorzeichen besitzen, betragsmäßig und vergrößern das Gesamtsignal. Gleichläufige Effekte hingegen wie z. B. störende Temperatureffekte oder pyroelektrische Effekte werden weitgehend ausgelöscht und damit kompensiert.

Neben einem bimorphen piezoelektrischen Biegesensor kann auch ein multimorpher Biegesensor verwendet werden, der aus mehreren Sensorpaaren mit abwechselnd antiparalleler Polarität zusammengesetzt ist.

Es kann vorgesehen sein, dass der Biegesensor wie eine Wippe auf einer Auflage, die an der Außenseite des Schädels des Patienten anbringbar ist, angeordnet ist. Bei dieser Ausgestaltung ist der Biegesensor auf einer Auflage angeordnet, die im Bereich einer Schädelnaht des Patienten angeordnet wird. Der bimorphe piezoelektrische Biegesensor kann sich unter der Wirkung der Volumenauslenkungen des Schädels wie eine Wippe um einen Drehpunkt bewegen. Die als Stirnband ausgebildete Haltevorrichtung erzeugt dabei über eine definierte Vorspannung eine für die Verbiegung nötige Gegen(lager)kraft auf den Biegesensor.

Eine alternative Ausgestaltung sieht vor, dass der Biegesensor an einem mittleren Abschnitt einer C-förmigen Halterung angeordnet ist, der zwischen zwei Endabschnitten angeordnet ist. Die C-förmige Halterung wird so auf dem Schädel eines Patienten angeordnet, dass sich die beiden Endabschnitte auf beiden Seiten der Schädelnaht befinden. Durch die pulsierende Volumenauslenkung des Schädels wird eine gegenläufige Bewegung der beiden Endabschnitte (Schenkel) der C-förmigen Halterung verursacht, die in einer Biegung des mittleren Abschnitts des Biegesensors resultieren, Die Anbringung des oder der Biegesensoren erfolgt immer so, dass keine oder nur vernachlässigbare Pulsationen durch externe Arterien oder Venen auf den Biegesensor übertragen werden. Das kann dadurch erreicht werden, dass pulsierende Arterien oder Venen keinen oder nur stark gedämpften mechanischen Kontakt zum Biegesensor oder zu dessen Befestigung, d. h. mit dem Stirnband, haben. Durch die C-förmige Halterung der erfindungsgemäßen Messvorrichtung lassen sich stark pulsierende externe Gefäße wie große Arterien zu diesem Zweck effektiv und einfach überbrücken. Dies kann zusätzlich auch an anderen Stellen durch Aussparungen in der Auflage erfolgen. Durch die Verwendung von Polsterauflagen aus Schaumstoff kann auch der Einfluss von kleineren und damit schwächer pulsierenden externen Gefäßen effektiv unter die Einflussgrenze gedämpft werden. Ein direkter bzw. nicht ausreichend gedämpfter Kontakt zu einer pulsierenden Arterie würde im Zeitsignal sofort anhand der typischen "arteriellen Kurvenform" und der deutlich höheren Amplitude sichtbar.

Eine weitere alternative Ausgestaltung sieht vor, dass die C-förmige Halterung mit einer Manschette oder einem Gurt wie ein Stirnband um den Kopf befestigt wird, so dass sich die pulsierende Volumenauslenkung auf die Zugspannung der Manschette oder des Gurts überträgt und diese ihrerseits über die Schenkel der C-förmigen Halterung eine entsprechende Biegung verursacht, die von dem Biegesensor erfasst wird. Über eine solche Manschette können kleinste Volumenauslenkungen des Schädels in Form einer Zugspannung an die C-Schenkel der Sensorhalterung übertragen werden. Dies resultiert ebenfalls in einer Biegung des C-Bogens, welche vom Biegesensor detektiert wird.

Durch ein derartiges Stirnband wird eine definierte Vorspannung erzeugt. Die Volumenveränderung des Schädels durch die Pulsationen des intrakraniellen Drucks rufen eine Biegung des piezoelektrischen Biegesensors hervor, die mittels der erfindungsgemäßen Messvorrichtung erfasst werden kann. Anhand der auf diese Weise gewonnenen Messwerte kann die Schädelinnendruckpulsation und damit deren Druckpulsform überwacht und Vitalzustandsgrößen wie der Schädelinnendruck aus der Druckpulsformcharakteristik und dessen Kennwerten berechnet werden. Die C-förmige Halterung kann auch umgekehrt auf dem Schädel des Patienten angeordnet werden, d. h. sodass sich die beiden Endabschnitte von dem Schädel weg erstrecken. Auch in diesem Fall wird durch das Stirnband eine Vorspannung erzeugt. Der bimorphe Biegesensor kann auf einer der beiden Seiten oder symmetrisch auf beiden Seiten des mittleren Abschnitts angebracht werden. Alternativ kann in diesem Beispiel wie auch in allen anderen Beispielen ein bimorpher Biegesensor in der neutralen Faser des mittleren Abschnitts angeordnet werden. Es ist auch möglich, dass mehrere piezoelektrische Biegesensoren im Inneren des mittleren Abschnitts symmetrisch zur neutralen Faser angeordnet sind. Auf der Außenseite des Schädels kann auch ein weiches, elastisches Auflagepolster angebracht werden, auf dem die C-förmige Halterung angebracht wird. Alternativ kann das Polster so am Stirnband befestigt sein, dass es schnell ausgewechselt werden kann.

Vorzugsweise kann die als Stirnband ausgebildete Haltevorrichtung eine Vorrichtung zum Erzeugen und Einstellen einer auf den Schädel des Patienten wirkenden Vorspannkraft aufweisen. Die Vorrichtung zum Erzeugen der Vorspannkraft kann vorzugsweise einen Kraftsensor oder einen Dehnungssensor aufweisen. Die Vorspannung kann von einem Benutzer über ein Handrad oder dergleichen oder alternativ mittels eines Motors eingestellt werden. Die Vorrichtung kann zu diesem Zweck ein linear-elastisches Dehnungselement wie z. B. eine Zugfeder enthalten. In einer weiteren Ausgestaltung kann sich dieses linear-elastische Dehnungselement nach der Einstellung einer konstanten Zugspannung bzgl. einer weiteren Auslenkung fixieren, d. h. blockieren lassen.

In diesem Zusammenhang wird es bevorzugt, dass die Vorrichtung zum Erzeugen der Vorspannkraft eine Anzeige für die Vorspannkraft oder einer dieser zugeordneten Spannung aufweist. Auf diese Weise kann durch den Benutzer eine bestimmte Vorspannkraft, die über das Stirnband auf den piezoelektrischen Biegesensor übertragen wird, eingestellt und kontrolliert werden.

Um die Benutzung der erfindungsgemäßen Messvorrichtung weiter zu vereinfachen, kann die Vorrichtung zum Erzeugen der Vorspannkraft zum automatischen Einstellen einer vorgegebenen Vorspannkraft ausgebildet sein. Dazu kann ein elektromechanischer oder ein pneumatischer Mechanismus vorhanden sein. Eine manuelle oder automatische Regelung der Vorspannkraft kann durch eine pneumatische Spannkrafteinstellung mittels eines integrierten Luftkissens in Kombination mit einem Luftdrucksensor erfolgen.

Optional kann das Stirnband zumindest über einen Teil seiner Länge eine Polsterung aufweisen. Die Polsterung kann auch aus mehreren separaten gepolsterten Auflagepunkten bestehen. Die Polsterung befindet sich an der Innenseite der als Stirnband ausgebildeten Haltevorrichtung. Die Polsterung kann aus einem elastischen Schaumstoff oder aus einem viskoseelastischen Memoryschaumstoff bestehen. Das Stirnband oder die Manschette kann vollständig anliegen oder nur an definierten Auflageflächen oder Auflagepunkten, um Störeinflüsse durch pulsierende Weichgewebe wie periphere Blutgefäße und Muskelaktivitäten zu minimieren, bzw. Verletzungen nicht zu berühren.

Es kann auch vorgesehen sein, dass die erfindungsgemäße Messvorrichtung einen oder mehrere Körperschallsensoren und/oder einen oder mehrere Beschleunigungssensoren, einen oder mehrere Lagesensoren und/oder einen oder mehrere Pulssensoren und/oder einen oder mehrere Blutdrucksensoren und/oder einen Temperatursensor aufweist und dass die Recheneinheit dazu ausgebildet ist, durch zumindest einen der erwähnten Sensoren äußere Störeinflüsse zu erfassen. Diese äußeren Störeinflüsse können nach der Erfassung rechnerisch eliminiert werden, sodass sie die Messung des Schädelinnendrucks nicht nachteilig beeinflussen.

Vorzugsweise kann der Biegesensor aus der als Stirnband ausgebildeten Halterung entnommen und ausgetauscht werden. Zur Befestigung des Sensors sind Haltevorrichtungen wie Aussparungen und/oder Halteklammern an der bevorzugten Sensorposition des Stirnbands oder der Manschette vorgesehen, so dass ein Formschluss und/oder ein Kraftschluss ermöglicht wird. Der Biegesensor kann jedoch auch auf das Stirnband oder die Manschette geklebt und/oder geschraubt sein. Das Stirnband kann nach einer Sterilisation wieder für einen anderen Patienten verwendet werden. Es ist auch möglich, dass das Stirnband unterschiedliche Positionen zur Anbringung des Biegesensors aufweist. Es ist auch möglich, dass mehrere Biegesensoren an dem Stirnband befestigt werden.

Eine Ausführung der erfindungsgemäßen Messvorrichtung sieht vor, dass der bimorphe Biegesensor und der Analogsignalverstärker in einem einzigen Bauteil integriert sind. Optional können auch die folgenden Komponenten in dem einzigen Bauteil integriert sein: A/D-Wandler, eine Sendeeinrichtung, eine Sende-Empfangs-Einrichtung zur drahtlosen Datenübertragung, eine Batterie, eine wiederaufladbare Batterie. Dadurch wird die Anzahl der Komponenten verringert und die Messvorrichtung benötigt lediglich einen kleinen Bauraum.

Es kann auch vorgesehen sein, dass die Messvorrichtung einen Datenlogger aufweist, der mit dem A/D-Wandler oder der Recheneinheit verbunden ist. Der Datenlogger speichert entweder die Messwerte des Biegesensors und/oder die daraus abgeleiteten Daten wie den Schädelinnendruck. Die in dem Datenlogger gespeicherten Daten können dadurch auch zu einem späteren Zeitpunkt ausgewertet werden. Die erfindungsgemäße Messvorrichtung kann somit auch als mobiles Gerät ausgebildet sein.

Die als Stirnband angeordnete Haltevorrichtung kann einen Energiespeicher aufweisen, vorzugsweise eine Batterie oder eine aufladbare Batterie, wodurch eine Nutzung als mobiles Gerät ermöglicht wird.

Weitergehende Einsatzmöglichkeiten ergeben sich, wenn der piezoelektrische Biegesensor und/oder der Analogsignalverstärker und/oder der A/D-Wandler mit einer Sendeeinrichtung oder einer Sende-Empfangseinrichtung zur drahtlosen Datenübertragung verbunden ist bzw. sind. In diesem Fall können vom Sensor erfasste Daten, gegebenenfalls nach der Verstärkung oder nach der Umwandlung in digitale Daten an einen Empfänger gesendet werden. Bei einer drahtlosen Datenübertragung benötigt die als Stirnband ausgebildete Haltevorrichtung keine Kabelverbindungen, wodurch die Handhabung vereinfacht und erleichtert wird.

Eine Variante der erfindungsgemäßen Messvorrichtung sieht vor, dass an dem Stirnband mehrere piezoelektrische Biegesensoren angeordnet sind. Dadurch wird die Messung der Schädelinnendruckpulsation und damit des Schädelinnendrucks an mehreren Stellen ermöglicht.

Die Erfindung wird nachfolgend anhand von Ausführungsbeispielen unter Bezugnahme auf die Zeichnungen erläutert. Die Zeichnungen sind schematische Darstellungen und zeigen:
- Fig. 1: einen normalen Verlauf und einen pathologischen Verlauf des Schädelinnendrucks über der Zeit;
- Fig. 2: die wesentlichen Komponenten einer erfindungsgemäßen Messvorrichtung;
- Fig. 3: ein weiteres Ausführungsbeispiel einer als Manschette ausgebildeten Haltevorrichtung;
- Fig. 4: ein Ausführungsbeispiel einer Haltevorrichtung mit mehreren Biegesensoren;
- Fig. 5: ein weiteres Ausführungsbeispiel einer Haltevorrichtung mit mehreren Biegesensoren;
- Fig. 6: eine Draufsicht einer als Manschette ausgebildeten Haltevorrichtung;
- Fig. 7: ein weiteres Ausführungsbeispiel einer als Manschette ausgebildeten Haltevorrichtung in einer Draufsicht;
- Fig. 8: ein ähnliches Ausführungsbeispiel einer Manschette wie Fig. 6;
- Fig. 9: ein Ausführungsbeispiel einer Manschette mit einem dehnbaren Band;
- Fig. 10: ein Ausführungsbeispiel einer Manschette mit einem dehnbaren Gummiband;
- Fig. 11: ein weiteres Ausführungsbeispiel einer Manschette;
- Fig. 12: eine Messvorrichtung ohne Manschette oder Band;
- Fig. 13: eine Messvorrichtung mit einer Manschette;
- Fig. 14a-14e: verschiedene Ausführungen einer C-förmigen Halterung;
- Fig. 15: einen auf einem Schädel platzierten Biegesensor in einer geschnittenen Ansicht;
- Fig. 16: einen auf einem Schädel platzierten Biegesensor in einer geschnittenen Ansicht,
- Fig. 17: eine Draufsicht einer auf einem Schädel platzierten Haltevorrichtung;
- Fig. 18: eine Ansicht der rechten Seite der in Fig. 17 gezeigten Haltevorrichtung, und
- Fig. 19: eine Ansicht der linken Seite der in Fig. 17 gezeigten Haltevorrichtung.

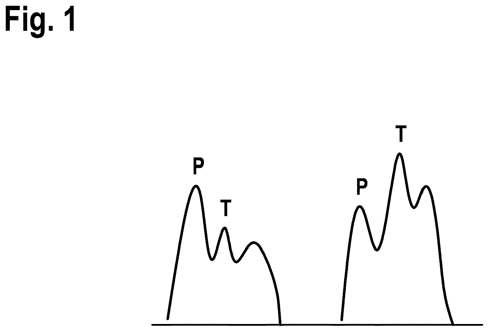

Im linken Teil von Fig. 1 ist qualitativ ein normaler Verlauf des Schädelinnendrucks dargestellt, im rechten Teil von Fig. 1 ist ein pathologischer Verlauf des Schädelinnendrucks gezeigt. Auf der horizontalen Achse ist die Zeit aufgetragen, auf der vertikalen Achse ist eine von dem Sensor erfasste elektrische Spannung aufgetragen. Anhand der Kurvenform des Spannung-zeit-Signals kann der Schädelinnendruck ermittelt werden. Kennwerte dafür sind zum Beispiel der Anstiegsquotient (U1-U0)/t0, die Anzahl an Peaks pro Herzzyklus, die typischerweise 3 bis 6 betragen kann. Die Auswertung kann auch anhand von Distanzen zwischen markanten Punkten mindestens eines Pulssignals und parallel mittels eines Elektrokardiogramms aufgezeichneten QRS-Komponenten oder eines am Kopf, Hals, Arm oder Finger abgegriffenen externen arteriellen Pulssignals erfolgen. Zur Auswertung kann auch eine Korrelation oder Korrektur mit der Pulsfrequenz oder der Atmungsfrequenz des Patienten erfolgen.

Bei kontinuierlicher Aufzeichnung zeigt der Schädelinnendruck (intracranial pressure, ICP) einen mehrgipfligen plussynchron-periodischen Verlauf: Der erste Gipfel P wird durch die arterielle Hauptdruckwelle verursacht. Ein zweiter Gipfel T entsteht durch das Füllen der zerebralen Arterien mit Blut und hängt ab von der intrakraniellen Compliance. Ein dritter Gipfel oder auch mehrere weitere Gipfel hängen mit diastolisch bedingten Pulsationen zusammen, z. B. mit dem Schließen der Aortenklappen.

Mit steigendem ICP nimmt T gegenüber P sowie die gesamte Pulsdruckamplitude zu, so dass die Kurvenform zunehmend pyramidenförmig wird. Aus dem dynamischen Verlauf ist somit ein Rückschluss auf einen erhöhten statischen Hirndruck möglich.

Durch die Erfassung der Auslenkung eines an einer Kopfmanschette, an einem Stirnband oder an einer Auflagefläche am Kopf angebrachten bimorphen piezoelektrischen Biegesensors aufgrund der pulssynchronen Schädelvolumenausdehnung durch den mit ca. 3-4 mmHg pulsierenden Schädelinnendruck kann der mittlere statische Schädelinnendruck (ICP) aus der Pulsform indirekt bestimmt werden. Zur Erhöhung der Genauigkeit dieses Verfahrens können absolut gemessene Blutdruckwerte hinzugezogen werden.

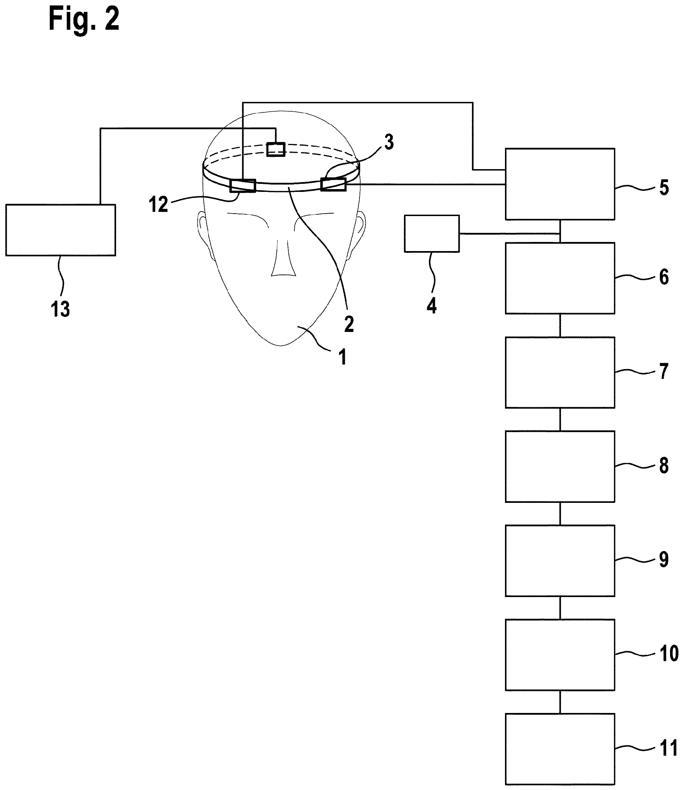

Wesentliche Komponenten der Messvorrichtung bzw. Schritte des Messverfahrens werden anhand von Fig. 2 erläutert. Am Kopf einer Person 1 ist eine Haltevorrichtung 2 angebracht, die als Stirnband oder Manschette ausgebildet ist. An dem Stirnband befindet sich ein piezoelektrischer Biegesensor 3, der an der Außenseite des Schädels der Person 1 lösbar angeordnet ist. Dem Biegesensor 3 ist ein Energiespeicher in Form einer aufladbaren Batterie 4 zugeordnet. Daneben umfasst die Messvorrichtung einen Analogsignalverstärker 5 mit einem Analogfilter. Daran schließt sich ein A/D-Wandler 6 an, der die analogen Signale in digitale Daten umwandelt. In einem Filter 7 erfolgt eine Filterung der digitalen Daten, eine Glättung und eine Datenreduktion. Die Messvorrichtung umfasst ferner eine Schnittstelle 8 zum Übertragen von Signalen bzw. Daten. Die Signale bzw. Daten können zum Beispiel an ein externes Gerät, eine Recheneinheit oder eine Auswerteeinheit übertragen werden. Anhand der Daten werden Kennwerte ermittelt, die in einem Kennwertspeicher 9 gespeichert werden. In einer Bewertungseinheit 10 findet eine Bewertung der Daten oder Kennwerte statt. Eine Anzeige 11 dient zum Ausgeben von Messdaten und anderen Informationen. Dazu zählen erfasste Messwerte, Signale, Kennwerte, eine Bewertung oder eine Warnung. An der Haltevorrichtung 2 ist zusätzlich ein Körperschallsensor 12 zur Erfassung von Störsignalen angebracht. Diese Weise können Störsignale, die durch externe Signalquellen verursacht werden, eliminiert werden. Bestandteil der Messvorrichtung ist auch eine Vorrichtung 13 zum Erzeugen einer Vorspannkraft. Mittels der Vorrichtung 13 kann eine auf den Biegesensor 3 ausgeübte definierte Vorspannkraft erzeugt werden.

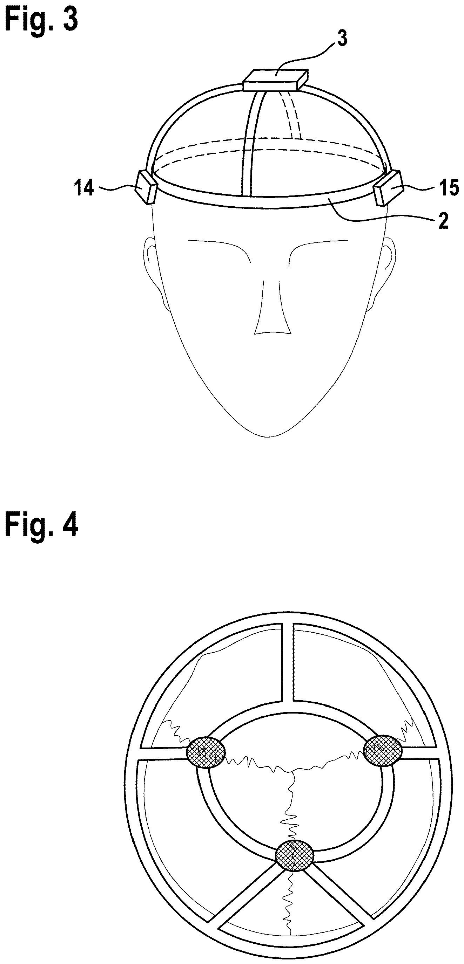

Fig. 3 zeigt ein Ausführungsbeispiel, bei dem die als Manschette ausgebildete Haltevorrichtung 2 neben den in Fig. 2 gezeigten Komponenten zusätzlich zwei weitere Biegesensoren 14, 15 aufweist. In dem auf dem Kopf angeordneten Biegesensor 3 befinden sich die weiteren in Fig. 2 erwähnten Komponenten wie ein Energiespeicher, ein Analogsignalverstärker, ein A/D-Wandler usw.

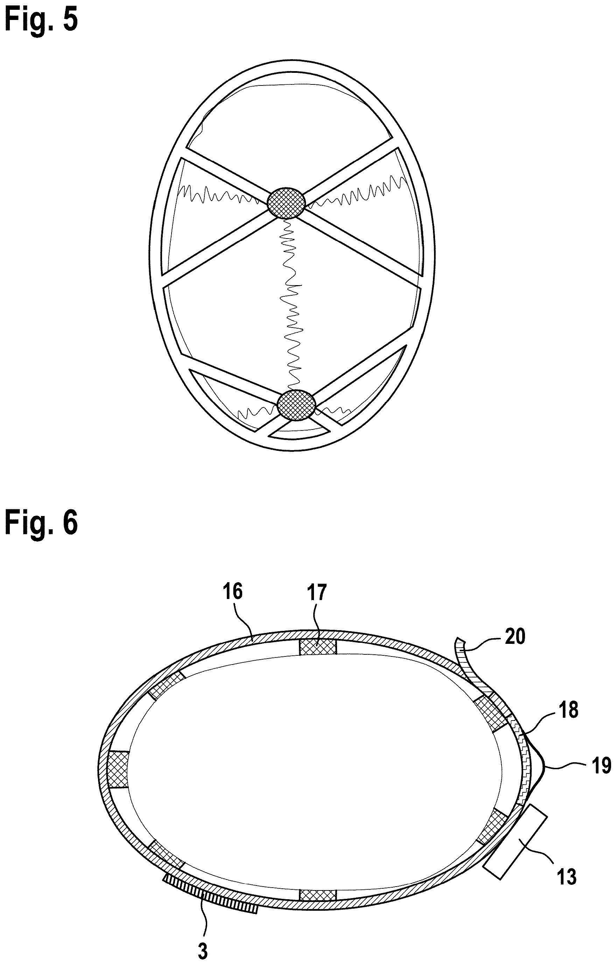

Die Fig. 4 und 5 zeigen weitere Beispiele von Haltevorrichtungen, die jeweils mehrere Biegesensoren aufweisen, die durch Aufbringen einer auf den Biegesensor wirkenden Vorspannkraft temporär auf dem Schädel eines Patienten fixiert sind.

Fig. 6 zeigt eine schematische Draufsicht einer als Manschette 16 ausgebildeten Haltevorrichtung, die an einem Schädel angebracht ist. Die Manschette 16 weist an ihrer Innenseite mehrere beabstandete Polster 17 auf, die jeweils eine Auflagefläche auf dem Schädel bilden. Zwischen benachbarten Polstern 17 kann ein Freiraum vorhanden sein, alternativ kann der Zwischenraum auch durch Schaumstoff ausgefüllt sein. An der Außenseite der Manschette 16 ist ein Biegesensor angeordnet. Die Manschette 16 umfasst auch die Vorrichtung 13 zum Erzeugen einer Vorspannkraft sowie ein als Gummiband ausgebildetes Dehnungselement 18. Die Dehnung wird durch einen Dehnungsbegrenzer 19 limitiert. Die Manschette 16 weist auch einen Klettverschluss 20 zum Befestigen eines freien Endes der Manschette 16 auf.

Bei einer alternativen Ausführung kann die Manschette an ihrer Innenseite mit einem viskoelastischen Memoryschaumstoff versehen sein. Dieser hat die Eigenschaft, dass er bei schneller Belastung, insbesondere bei einem schnellen Stoß, hart wird.

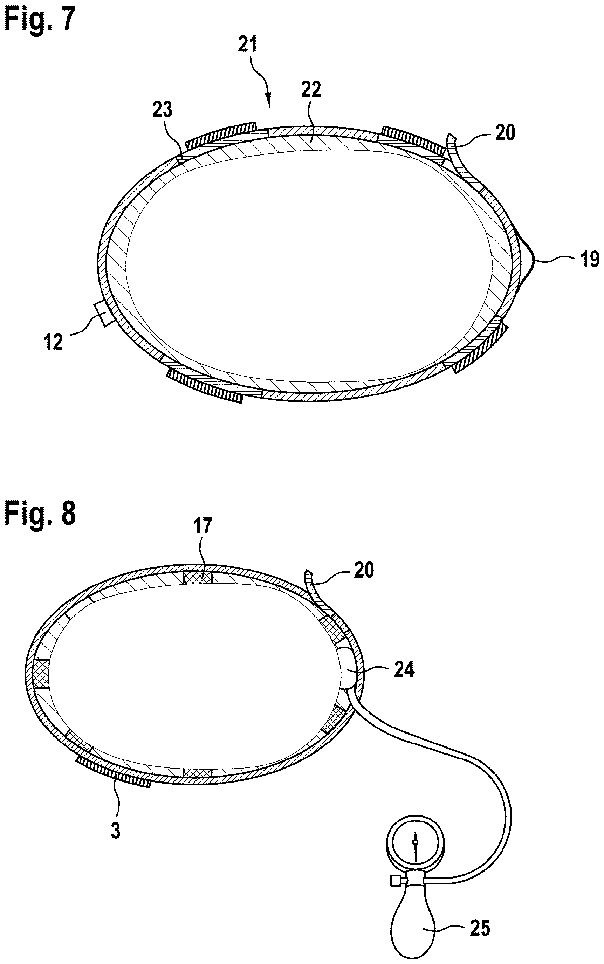

Fig. 7 zeigt ein weiteres Ausführungsbeispiel einer als Manschette 21 ausgebildeten Haltevorrichtung. Die Manschette 21 ist aus einem elastischen, d. h. dehnbaren Material hergestellt. In Übereinstimmung mit dem vorangehenden Ausführungsbeispiel weist die Manschette 21 den Klettverschluss 20 und den Dehnungsbegrenzer 19 auf. An der Innenseite der Manschette 21 befindet sich ein viskoelastischer Memoryschaumstoff 22 als Polster. An der Außenseite der Manschette 21 sind insgesamt vier über den Umfang verteilte Biegesensoren 3 angeordnet. Jeder Biegesensor 3 ist auf einer flexiblen Unterlage 23 angebracht. Zusätzlich ist ein Körperschallsensor 12 an der Außenseite der Manschette 21 angeordnet.

Fig. 8 zeigt ein Ausführungsbeispiel, das ähnlich wie die in Fig. 6 gezeigte Manschette 16 ausgebildet ist. Die Manschette weist neben den Polstern 17, die Auflageflächen bilden und dem Biegesensor 3 ein Luftkissen 24 auf, das durch eine manuell betätigte Pumpe 25 aufgepumpt werden kann.

Die beschriebenen Ausführungsbeispiele zeigen jeweils geschlossene Manschetten, die sich über den gesamten Umfang des Schädels eines Patienten erstrecken. Eine Manschette kann sich jedoch auch lediglich über einen Teil des Umfangs des Schädels erstrecken und geklemmt werden. Die Manschette kann dazu aus einem flexiblen Material, einem biegbaren Material oder aus einem federelastischen Material bestehen.

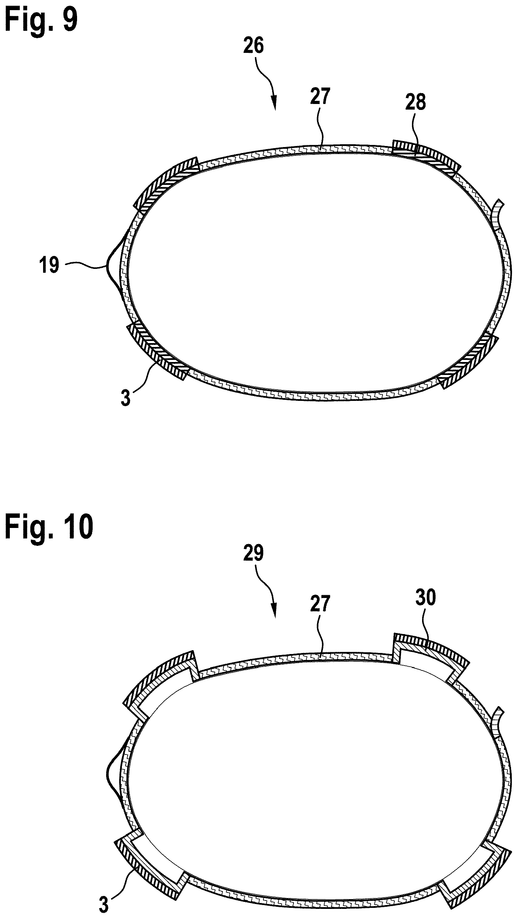

Fig. 9 zeigt ein Ausführungsbeispiel einer Manschette 26 mit einem dehnbaren Gummiband 27, das sich über den ganzen Umfang eines Schädels erstreckt. An der Außenseite des Gummiband 27 befinden sich mehrere Biegesensoren 3, die über ein biegeflexibles Element 28 mit einem Polster an dem Schädel eines Patienten angebracht sind. Des Gummiband 27 weist auch einen Dehnungsbegrenzer 19 auf.

Bei einer abgewandelten Ausführung kann anstelle eines dehnbaren Gummibands ein nicht dehnbarer Spanngurt verwendet werden. In diesem Fall ist ein kurzes den Element erforderlich, um die Manschette mit einer gewissen Vorspannung an einem Schädel anzubringen.

Fig. 10 zeigt ein Ausführungsbeispiel einer Manschette 29 mit einem dehnbaren Gummiband 27 und mehreren Biegesensoren 3, die jeweils an der Außenseite einer C-förmigen Halterung 30 angeordnet sind. Eine C-förmige Halterung 30 umfasst einen mittleren Abschnitt und zwei sich davon senkrecht erstreckende Endabschnitte. Die Endabschnitte der C-förmigen Halterungen 30 weisen zum Schädel hin. Die C-förmigen Halterungen 30 sind biegeflexibel (biegeweich) und werden so in Umfangsrichtung auf einem Schädel eines Patienten angeordnet, dass sie eine Schädelnaht überdecken. Eine pulsierende Dehnung des Schädels kann mittels der Biegesensoren 3 erfasst werden.

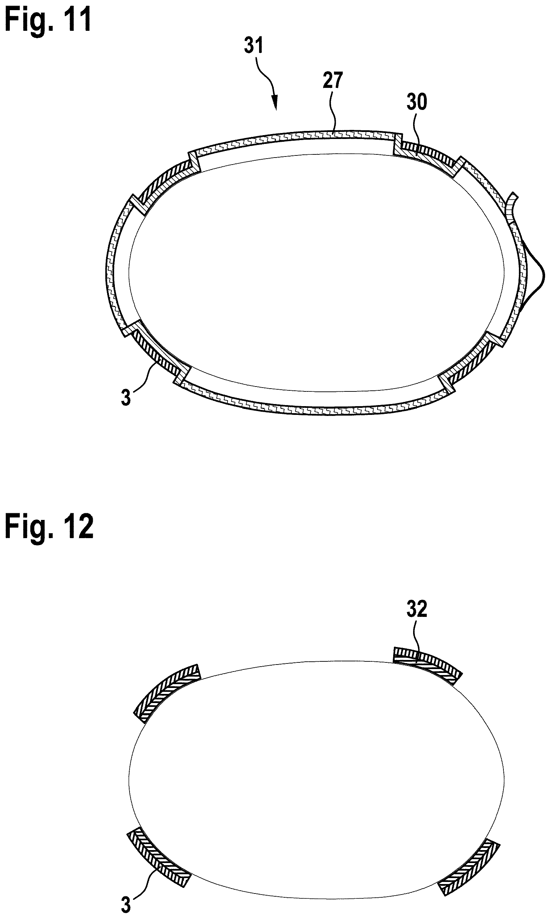

Fig. 11 zeigt ein Ausführungsbeispiel einer Manschette 31, die ähnlich wie das in Fig. 10 gezeigte Ausführungsbeispiel ausgebildet ist. An der Manschette 31 sind insgesamt vier C-förmige Halterungen 30 angeordnet, deren Endabschnitte vom Schädelweg weisen. An der Außenseite jeder C-förmigen Halterung 30 befindet sich jeweils ein Biegesensor 3.

Fig. 12 zeigt ein Ausführungsbeispiel einer Messvorrichtung, bei der die Haltevorrichtung als biegeflexibles Element 32 ausgebildet ist. An der Außenseite des biegeflexiblen Elements 32 ist ein Biegesensor 3 angeordnet. Insgesamt sind über den Umfang des Schädels vier derartige Biegesensoren 3 vorhanden. Die biegeflexiblen Elemente 32 sind an den Schädel geklebt, eine Manschette oder ein Band wird bei dieser Ausführung nicht benötigt.

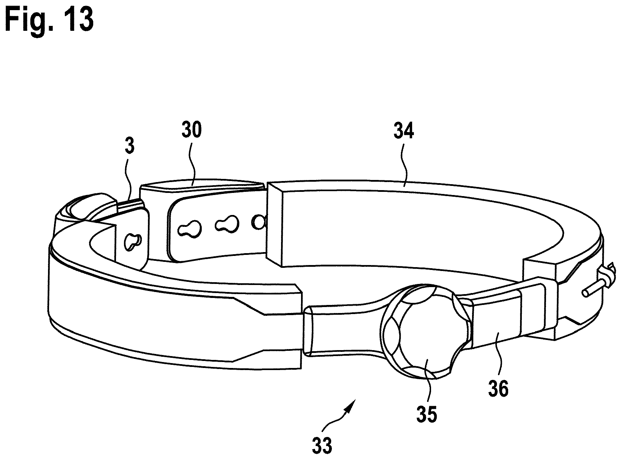

Fig. 13 zeigt eine Messvorrichtung mit einer Manschette 33, einer C-förmigen Halterung 30 und einem piezoelektrischen Biegesensor 3.an der Innenseite der Manschette 33 befinden sich Polster 34 aus Schaumstoff. An der dem piezoelektrischen Biegesensor entgegengesetzten Seite ist eine Vorrichtung 13 zum Erzeugen einer Vorspannkraft mit einer Einstellschraube 35 vorhanden. Die Vorrichtung 13 umfasst eine Anzeige 36 zum Anzeigen der Vorspannkraft.

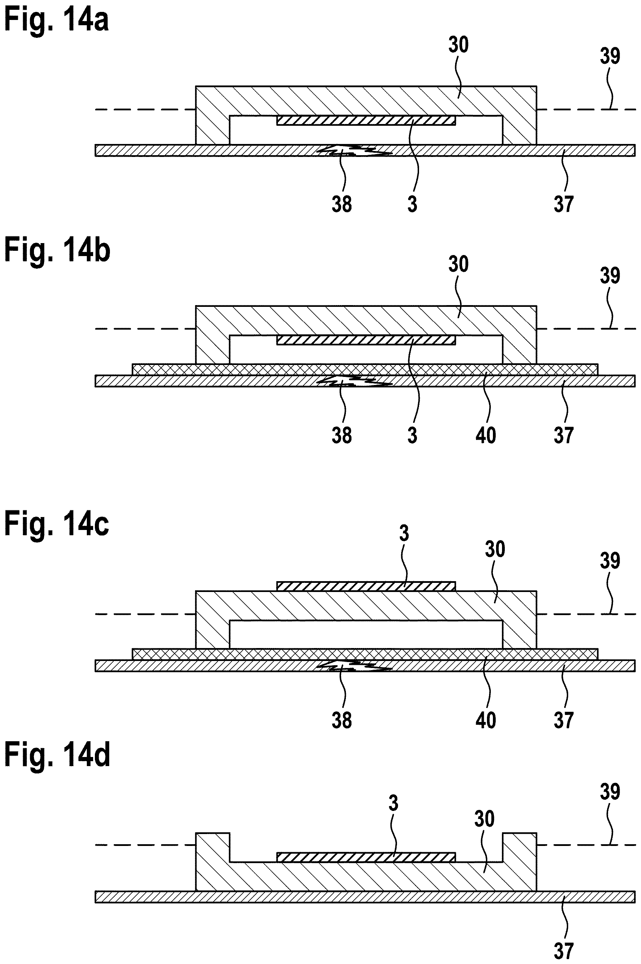

Fig. 14a bis 14e zeigen verschiedene Ausführungen einer C-förmigen Halterung.

In Fig. 14a erkennt man, dass die C-förmige Halterung 30 mit ihren Endabschnitten an der Außenseite 37 eines Schädels eines Patienten angeordnet ist. Der Biegesensor 3 befindet sich an der Innenseite der C-förmigen Halterung 30, die so angeordnet ist, dass sie eine Schädelnaht 38 überquert. Ein Band 39 dient zum Fixieren der C-förmigen Halterung 30. Das Band 39 kann starr, biegsam, flexibel oder dehnbar sein.

Fig. 14b ist eine ähnliche Ansicht wie Fig. 14a, wobei zwischen der Außenseite 37 des Schädels und der C-förmigen Halterung 30 ein weiches elastisches Polster 40 angeordnet ist.

Fig. 14c ist eine ähnliche Ansicht wie Fig. 14b, wobei der Biegesensor 3 an der Außenseite der C-förmigen Halterung 3 angeordnet ist.

Fig. 14d zeigt eine Ausführung, bei der die C-förmige Halterung 30 mit ihrem mittleren Abschnitt auf der Außenseite 37 eines Schädels aufliegt. Die Endabschnitte der C-förmigen Halterung 30 stehen somit von der Außenseite 37 des Schädels ab. Der Biegesensor 3 befindet sich auf der Außenseite der C-förmigen Halterung 30, die von dem Band 39 gehalten wird. Optional kann zwischen der Außenseite 37 des Schädels und der C-förmigen Halterung 30 ein weiches elastisches Polster angeordnet sein.

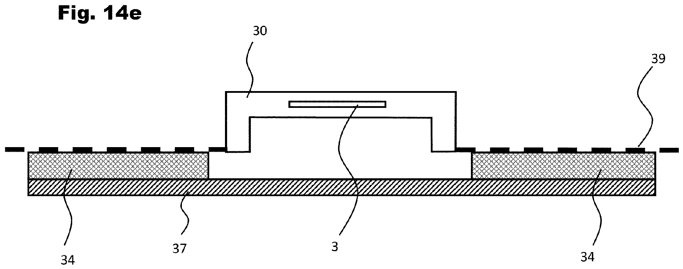

Fig. 14e zeigt eine Ausführung, bei der der Biegesensor 3 in der Haltevorrichtung 30 integriert ist. Er befindet sich im Inneren des mittleren Abschnitts der Haltevorrichtung 30, die an beliebiger Stelle an der Außenseite 37 des Kopfes eines Patienten anbringbar ist, jedoch so, dass Arterien dabei überbrückt werden. Zwischen dem Schädel und den abgewinkelten Endabschnitten befindet sich ein Polster 34. Durch einen Gurt oder ein Band 39, das an beiden Seiten der Endabschnitte der C-förmigen Halterung angebracht ist, kann eine Vorspannkraft eingeleitet werden.

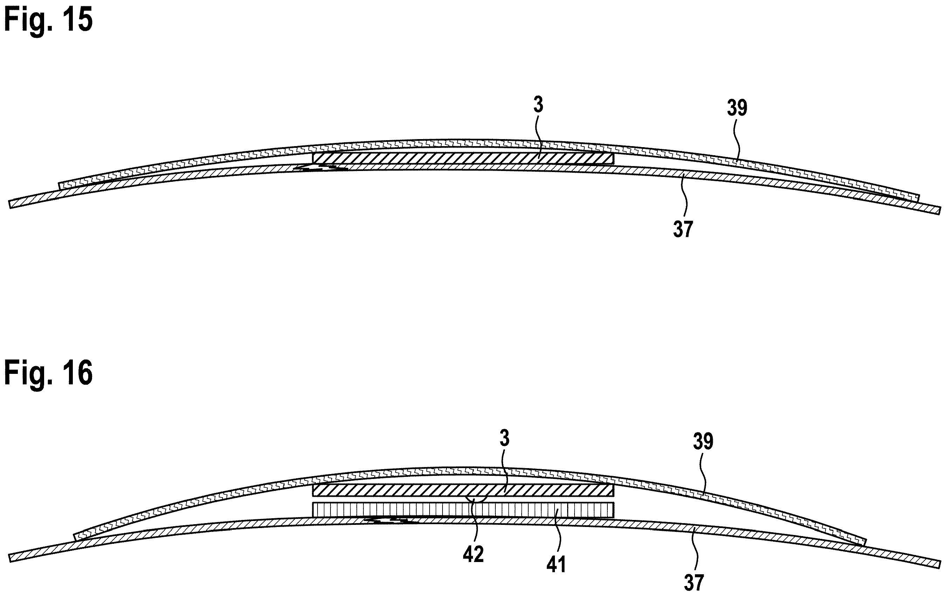

Fig. 15 ist eine geschnittene Ansicht und zeigt schematisch einen bimorphen piezoelektrischen Biegesensor 3, der auf der Außenseite 37 eines Schädels platziert ist. Das Band 39 dient zur Erzeugung einer Vorspannung. Ein Ende des Biegesensors 3 ist auf einer Schädelnaht angeordnet, dabei kann es sich z. B. um die Sutura coronalis, die Sutura sagittalis oder die Sutura lambdoidea handeln.

Fig. 16 ist eine geschnittene Ansicht und zeigt einen bimorphen piezoelektrischen Biegesensor 3, an dessen Unterseite sich in der Mitte ein konvexer, nach außen gewölbter Vorsprung 42 befindet, der sich auf einer starren Auflage 41 abstützt. Die Auflage 41 überdeckt eine Schädelnaht. Eine auf den Biegesensor 3 wirkende Vorspannung wird durch das Band 39 erzeugt. Durch den Vorsprung 42 ist der Biegesensor 3 wie eine Wippe gelagert, Volumenänderungen des Schädels können sich über das Band auf den Sensor übertragen, diesen durchbiegen und somit erfasst werden.

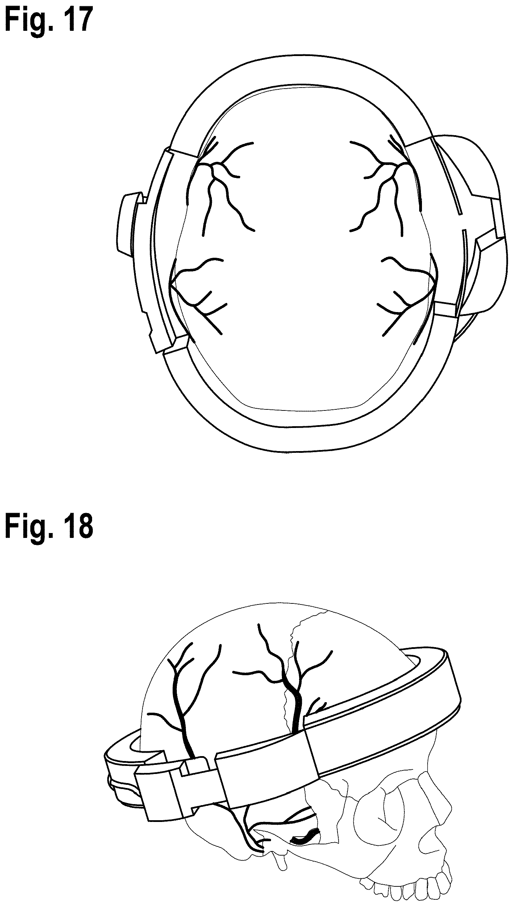

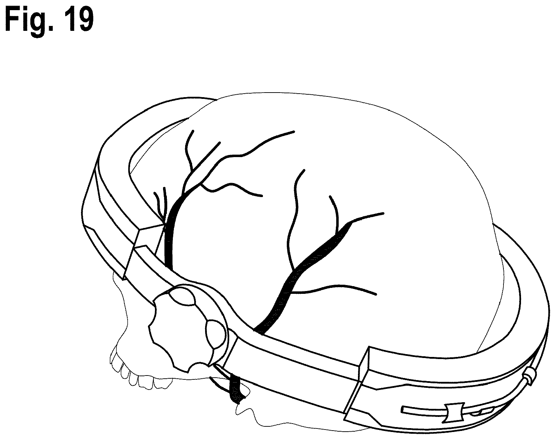

Fig. 17 ist eine Draufsicht und zeigt eine auf einem Schädel angeordnete Haltevorrichtung. Fig. 18 zeigt die rechte Seite der in Fig. 17 gezeigten Haltevorrichtung mit der C-förmigen Halterung und Fig. 19 zeigt die linke Seite der in Fig. 17 gezeigten Haltevorrichtung mit der Feststellvorrichtung, mit der eine Vorspannkraft erzeugt werden kann.

In den Fig. 17 - 19 erkennt man, dass die C-förmige Halterung der Haltevorrichtung mit ihren Endabschnitten an der Außenseite eines Schädels eines Patienten angeordnet ist. Der bimorphe Biegesensor befindet sich symmetrisch zur neutralen Faser des mittleren Abschnitts der C-förmigen Halterung im Inneren dieser Halterung, die so am Schädel angebracht wird, dass sie die Arteria Carotis Externa überbrückt. Ein Band dient zur Übertragung der durch Hirninnendruckpulsation verursachten Volumenauslenkung des Schädels in eine Biegung der C-förmigen Halterung. Das Band dieser Haltevorrichtung ist starr in Zugrichtung, flexibel in Biegerichtung und zum Schädel hin gepolstert. Auf der gegenüberliegenden linken Seite befindet sich die Feststellvorrichtung, die zum Einstellen der Vorspannung dient, sowie eine Anzeige für die Vorspannung. Auch die Feststellvorrichtung ist C-förmig ausgebildet und überbrückt die Arteria Carotis Externa, um dadurch hervorgerufene Störeinflüsse zu vermeiden.

Durch eine gezielte mechanische Ankopplung des Bands oder der Polsterung an eine oder mehrere Arterien (z. B. Arteria Carotis), kann die Anordnung auch zur Messung der Blutdruckpulsation und damit zur Blutdruckmessung am Kopf verwendet werden. Das Signal der externen Blutdruckpulsation ist in dieser Konfiguration deutlich höher als das Signal durch die Schädelauslenkung, verursacht durch die Hirninnendruckpulsation. Eine solche "vorübergehende" Ankopplung kann durch Verdrehung der Kopfmanschette, d. h. des Bands, um 90° erfolgen, so dass die Arteria Carotis unter der Auflageflächen des Bands liegt. Gleiches könnte jedoch auch ohne Drehung des Bands durch das Einfügen eines Schaumstoffs unter den Feststellmechanismus und/oder die C-förmige Halterung erfolgen. Vorteilhafterweise enthält die als Band ausgebildete Kopfmanschette an der Position der C-förmigen Halterung und/oder der Feststellvorrichtung ein Kopplungselement, welches sich reversibel an die Arteria Carotis ankoppeln lässt. Dies kann beispielsweise über ein Gewinde oder einen ausklappbaren Koppelmechanismus analog wie ein Sprung- bzw. Schnappschalter erfolgen. Die Ankopplungskraft lässt sich mit der vorhandenen Feststellvorrichtung einjustieren, vorzugsweise auf einen Wert, bei dem das System kalibriert wurde.

Die beschriebenen Merkmale der Messvorrichtung können in beliebiger Weise miteinander kombiniert werden.

### Bezugszeichenliste

- 1: Kopf
- 2: Haltevorrichtung
- 3: Biegesensor
- 4: Energiespeicher
- 5: Analogsignalverstärker
- 6: A/D-Wandler
- 7: Filter
- 8: Schnittstelle
- 9: Kennwertspeicher
- 10: Bewertungseinheit
- 11: Anzeige
- 12: Körperschallsensor
- 13: Vorrichtung zum Erzeugen einer Vorspannkraft
- 14: Biegesensor
- 15: Biegesensor
- 16: Manschette
- 17: Polster
- 18: Dehnungselement
- 19: Dehnungsbegrenzer
- 20: Klettverschluss
- 21: Manschette
- 22: Memoryschaumstoff
- 23: Unterlage
- 24: Luftkissen
- 25: Pumpe
- 26: Manschette
- 27: Gummiband
- 28: biegeflexibles Element
- 29: Manschette
- 30: C-förmige Halterung
- 31: Manschette
- 32: biegeflexibles Element
- 33: Manschette
- 34: Polster
- 35: Einstellschraube
- 36: Anzeige
- 37: Außenseite
- 38: Schädelnaht
- 39: Band
- 40: Polster
- 41: Auflage
- 42: Vorsprung

## Patentansprüche

1. Messvorrichtung zum nicht-invasiven Erfassen der Hirninnendruckpulsation eines Patienten (1), umfassend:
- eine Haltevorrichtung (2), die kraft- und/oder formschlüssig an der Außenseite des Schädels des Patienten (1) lösbar anbringbar ist
- wenigstens einen zwei Sensorschichten mit antiparalleler Polarität, die symmetrisch um die neutrale Faser angeordnet sind, aufweisenden bimorphen Biegesensor (3, 14, 15), der in oder an der Haltevorrichtung (2) angeordnet ist, wobei bei Biegung in eine Richtung eine der Biegesensorschichten gedehnt wird, während die andere gleichermaßen gestaucht wird, wobei sich die Signale bei gegenläufigen Belastungen der Biegesensorschichten addieren,
- einen Analogsignalverstärker (5) zum Verstärken der von dem bimorphen Biegesensor (3, 14, 15) gelieferten Messdaten,
- einen A/D-Wandler (6) zum Umwandeln der analogen Messdaten in digitale Daten, und
- eine Recheneinheit zur Vorverarbeitung der Daten und Berechnung von Parametern aus dem Hirninnendruckpulsationverlauf, welche mit Vitalzustandsgrößen korrelieren, anhand der digitalen Daten.

2. Messvorrichtung nach Anspruch 1, wobei die Haltevorrichtung (2) als Stirnband oder Kopfmanschette ausgebildet ist und/oder eine Anzeige (11) aufweist, um eine Messkurve, einen berechneten Parameter und einen zugehörigen zeitlichen Verlauf anzuzeigen.

3. Messvorrichtung nach Anspruch 1 oder 2, wobei der bimorphe Biegesensor (3, 14, 15) ein piezoelektrischer bimorpher Biegesensor ist.

4. Messvorrichtung nach einem der vorangehenden Ansprüche, wobei der Biegesensor (3, 14, 15) wie eine Wippe auf einer Auflage, die an der Außenseite des Schädels des Patienten anbringbar ist, angeordnet und um einen Drehpunkt bewegbar ist.

5. Messvorrichtung nach einem der Ansprüche 1 bis 3, wobei der Biegesensor (3, 14, 15) an oder in einem mittleren Abschnitt einer C-förmigen Halterung (30) angeordnet ist, der zwischen zwei Endabschnitten angeordnet ist.

6. Messvorrichtung nach einem der Ansprüche 2 bis 5, wobei die als Stirnband ausgebildete Haltevorrichtung (2) eine Vorrichtung (13) zum Erzeugen und Einstellen einer auf den Schädel des Patienten wirkenden Vorspannkraft aufweist, wobei die Vorrichtung (13) vorzugsweise einen Kraftsensor oder einen Dehnungssensor aufweist.

7. Messvorrichtung nach Anspruch 6, wobei die Vorrichtung (13) zum Erzeugen der Vorspannkraft eine Anzeige für die Vorspannkraft oder eine dieser zugeordneten Spannung aufweist.

8. Messvorrichtung nach Anspruch 6 oder 7, wobei die Vorrichtung (13) zum Erzeugen der Vorspannkraft zum automatischen Einstellen einer vorgegebenen Vorspannkraft ausgebildet ist und vorzugsweise einen elektromechanischen oder einen pneumatischen Mechanismus aufweist.

9. Messvorrichtung nach einem der vorangehenden Ansprüche, wobei eine als Stirnband ausgebildete Haltevorrichtung (2) zumindest über einen Teil ihrer Länge eine Polsterung (17, 40) aufweist.

10. Messvorrichtung nach einem der vorangehenden Ansprüche, wobei die Vorrichtung einen oder mehrere der folgenden Sensoren aufweist: Körperschallsensor (12), Beschleunigungssensor, Lagesensor, externer Pulssensor, externer Blutdrucksensor, Temperatursensor, und wobei die Recheneinheit dazu ausgebildet ist, durch wenigstens einen der genannten Sensoren erfasste äußere Störeinflüsse oder Zustände zu erfassen und gegebenenfalls Störeinflüsse zu korrigieren.

11. Messvorrichtung nach einem der vorangehenden Ansprüche, wobei der Biegesensor (3, 14, 15) aus der als Stirnband ausgebildeten Halterung (2) entnehmbar und austauschbar ist.

12. Messvorrichtung nach einem der vorangehenden Ansprüche, wobei die Messvorrichtung einen Datenlogger aufweist, der mit dem A/D-Wandler (6) oder der Recheneinheit verbunden ist.

13. Messvorrichtung nach einem der vorangehenden Ansprüche, wobei die als Stirnband angeordnete Haltevorrichtung (2) einen Energiespeicher (4) aufweist, vorzugsweise eine Batterie oder eine aufladbare Batterie.

14. Messvorrichtung nach einem der vorangehenden Ansprüche, wobei der piezoelektrischen Biegesensor (3, 14, 15) und/oder der Analogsignalverstärker (5) und/oder der A/D-Wandler (6) mit einer Sendeeinrichtung oder einer Sende-Empfangseinrichtung zur drahtlosen Datenübertragung verbunden sind.

15. Messvorrichtung nach einem der vorangehenden Ansprüche, wobei der bimorphe Biegesensor (3, 14, 15) und der Analogsignalverstärker (5) und der A/D-Wandler (6) und/oder die gegebenenfalls vorhandene Sendeeinrichtung und/oder die gegebenenfalls vorhandene Sende-Empfangseinrichtung zur drahtlosen Datenübertragung und/oder die gegebenenfalls vorhandene Batterie oder aufladbare Batterie in einem einzigen Bauteil integriert sind.

16. Messvorrichtung nach einem der vorangehenden Ansprüche, wobei an einer als Stirnband ausgebildeten Haltevorrichtung (2) mehrere piezoelektrische Biegesensoren (3, 14, 15) angeordnet sind.

17. Messvorrichtung nach einem der vorangehenden Ansprüche, wobei der Biegesensor (3, 4, 15) ein aus mehreren Sensorpaaren mit abwechselnd antiparalleler Polarität zusammengesetzter multimorpher Biegesensor ist.

## Claims

1. Measuring device for non-invasively detecting the intracranial pressure pulsation of a patient (1), comprising:
- a holding device (2) which can be detachably attached to the outside of the patient's (1) skull in a force-locking and/or form-locking manner,
- at least one bimorph bending sensor (3, 14, 15) having two sensor layers with antiparallel polarity arranged symmetrically around the neutral fibre, which is arranged in or on the holding device (2), wherein upon bending in one direction one of the bending sensor layers is stretched while the other is equally compressed, the signals adding up when the bending sensor layers are loaded in opposite directions,
- an analog signal amplifier (5) for amplifying the measurement data supplied by the bimorph bending sensor (3, 14, 15),
- an A/D converter (6) for converting the analog measurement data into digital data, and
- a computing unit for preprocessing the data and calculating parameters from the intracranial pressure pulsation curve, which correlate with vital state variables on basis of the digital data.

2. Measuring device according to claim 1, wherein the holding device (2) is formed as a headband or head cuff and/or has a display (11) to display a measurement curve, a calculated parameter and an associated time course.

3. Measuring device according to claim 1 or 2, wherein the bimorph bending sensor (3, 14, 15) is a piezoelectric bimorph bending sensor.

4. Measuring device according to any of the preceding claims, wherein the bending sensor (3, 14, 15) is arranged like a rocker on a support which can be attached to the outside of the patient's skull and is movable about a pivot point.

5. Measuring device according to any one of claims 1 to 3, wherein the bending sensor (3, 14, 15) is arranged at or in a middle portion of a C-shaped holder (30) arranged between two end sections.

6. Measuring device according to any one of claims 2 to 5, wherein the holding device (2) designed as a headband has a device (13) for generating and adjusting a pretensioning force acting on the patient's skull, the device (13) preferably having a force sensor or a strain sensor.

7. Measuring device according to claim 6, wherein the device (13) for generating the pretensioning force comprises an indicator for the pretensioning force or a voltage associated therewith.

8. Measuring device according to claim 6 or 7, wherein the device (13) for generating the pretensioning force is designed for automatically setting a predetermined pretensioning force and preferably comprises an electromechanical or a pneumatic mechanism.

9. Measuring device according to any of the preceding claims, wherein a holding device (2), which is in the form of the headband has pads (17, 40) over at least part of its length.

10. Measuring device according to any of the preceding claims, wherein the device comprises one or more of the following sensors: structure-borne sound sensor (12), acceleration sensor, position sensor, external pulse sensor, external blood pressure sensor, temperature sensor, and wherein the computing unit is designed to detect external disturbing influences or conditions detected by at least one of said sensors and, if necessary, to correct disturbing influences.

11. Measuring device according to any of the preceding claims, wherein the bending sensor (3, 14, 15) is removable and replaceable from the holder (2) formed as a headband.

12. Measuring device according to any of the preceding claims, wherein the measuring device comprises a data logger connected to the A/D converter (6) or the computing unit.

13. Measuring device according to any of the preceding claims, wherein the holding device (2) arranged as a headband has an energy storage device (4), preferably a battery or a rechargeable battery.

14. Measuring device according to any of the preceding claims, wherein the piezoelectric bending sensor (3, 14, 15) and/or the analog signal amplifier (5) and/or the A/D converter (6) are connected to a transmitting device or a transmitting-receiving device for wireless data transmission.

15. Measuring device according to any of the preceding claims, wherein the bimorph bending sensor (3, 14, 15) and the analog signal amplifier (5) and the A/D converter (6) and/or the transmitting device, if any, and/or the transmitting-receiving device for wireless data transmission, if any, and/or the battery or rechargeable battery, if any, are integrated in a single component.

16. Measuring device according to any of the preceding claims, wherein a plurality of piezoelectric bending sensors (3, 14, 15) are arranged at a holding device (2) designed as a headband.

17. Measuring device according to any of the preceding claims, wherein the bending sensor (3, 14, 15) is a multimorphic bending sensor composed of several pairs of sensors with alternating antiparallel polarity.

## Revendications

1. Dispositif de mesure pour la saisie non invasive de la pulsation de pression intracrânienne d'un patient (1) comprenant :
- un dispositif de retenue (2), qui peut être monté de manière amovible par adhérence et/ou par complémentarité de forme sur la face externe du crâne du patient,
- au moins un capteur de flexion (3, 14, 15) bimorphe comportant deux couches de capteur de polarité antiparallèle disposées symétriquement autour de la fibre neutre, ledit capteur étant disposé dans ou sur le dispositif de retenue (2), en ce que, en cas de flexion dans une direction, l'une des couches de capteur de flexion est étirée, tandis que l'autre est de la même manière comprimée, les signaux s'additionnant en cas de charges en sens contraire des couches de capteur de flexion,
- un amplificateur de signaux analogiques (5) pour amplifier les données de mesure fournies par le capteur de flexion (3, 14, 15) bimorphe,
- un convertisseur A/N (6) pour convertir les données de mesure analogiques en données numériques, et
- une unité de calcul pour le prétraitement des données et le calcul de paramètres à partir de la courbe de pulsation de pression intracrânienne qui sont en corrélation avec des grandeurs d'état vital, à l'aide des données numériques.

2. Dispositif de mesure selon la revendication 1, en ce que le dispositif de retenue (2) est conçu sous forme de bandeau ou de serre-tête et/ou comporte un affichage (11) permettant d'afficher une courbe de mesure, un paramètre calculé et un tracé dans le temps correspondant.

3. Dispositif de mesure selon la revendication 1 ou 2, en ce que le capteur de flexion bimorphe (3, 14, 15) est un capteur de flexion bimorphe piézoélectrique.

4. Dispositif de mesure selon l'une des revendications précédentes, en ce que le capteur de flexion (3, 14, 15) est disposé comme une bascule sur un support qui peut être fixé sur la face externe du crâne du patient, et est mobile autour d'un point de rotation.

5. Dispositif de mesure selon l'une des revendications 1 à 3, en ce que le capteur de flexion (3, 14, 15) est disposé sur ou dans une section centrale d'un support en forme de C (30), laquelle est disposée entre deux sections d'extrémité.

6. Dispositif de mesure selon l'une des revendications 2 à 5, en ce que le dispositif de retenue (2) conçu sous forme de bandeau comporte un dispositif (13) permettant la génération et le réglage d'une force de précontrainte agissant sur le crâne du patient, en ce que le dispositif (13) comporte de préférence un capteur de force ou un capteur d'allongement.

7. Dispositif de mesure selon la revendication 6, en ce que le dispositif (13) permettant la génération de la force de précontrainte comporte un affichage pour la force de précontrainte ou une tension qui lui est associée.

8. Dispositif de mesure selon la revendication 6 ou 7, en ce que le dispositif (13) permettant la génération de la force de précontrainte est conçu pour le réglage automatique d'une force de précontrainte prédéfinie et comporte de préférence un mécanisme électromécanique ou pneumatique.

9. Dispositif de mesure selon l'une des revendications précédentes, en ce qu'un dispositif de retenue (2) conçu sous forme de bandeau comporte un coussin (17, 40) au moins sur une partie de sa longueur.

10. Dispositif de mesure selon l'une des revendications précédentes, en ce que le dispositif comporte un ou plusieurs des capteurs suivants : capteur acoustique corporel (12), capteur d'accélération, capteur de position, capteur de pouls externe, capteur de pression artérielle externe, capteur de température, et l'unité de calcul étant conçue pour détecter des influences perturbatrices ou états extérieurs détectés par au moins l'un desdits capteurs et, le cas échéant, pour corriger des influences perturbatrices.

11. Dispositif de mesure selon l'une des revendications précédentes, en ce que le capteur de flexion (3, 14, 15) peut être retiré et remplacé du dispositif de retenue (2) conçu sous forme de bandeau.

12. Dispositif de mesure selon l'une des revendications précédentes, en ce que le dispositif de mesure comporte un enregistreur de données qui est relié au convertisseur A/N (6) ou à l'unité de calcul.

13. Dispositif de mesure selon l'une des revendications précédentes, en ce que le dispositif de retenue (2) disposé sous forme de bandeau comporte un accumulateur d'énergie (4), de préférence une batterie ou une batterie rechargeable.

14. Dispositif de mesure selon l'une des revendications précédentes, en ce que le capteur de flexion (3, 14, 15) piézoélectrique et/ou l'amplificateur de signaux analogiques (5) et/ou le convertisseur A/N (6) sont reliés à une installation émettrice ou à une installation émettrice-réceptrice pour la transmission de données sans fil.

15. Dispositif de mesure selon l'une des revendications précédentes, en ce que le capteur de flexion (3, 14, 15) bimorphe et l'amplificateur de signaux analogiques (5) et le convertisseur A/N (6) et/ou l'installation émettrice éventuellement existante et/ou l'installation émettrice-réceptrice éventuellement existante pour la transmission de données sans fil et/ou la batterie éventuellement existante ou la batterie rechargeable sont intégrés dans un seul composant.

16. Dispositif de mesure selon l'une des revendications précédentes, en ce que plusieurs capteurs de flexion (3, 14 ,15) piézoélectriques sont disposés sur un dispositif de retenue (2) conçu sous forme de bandeau.

17. Dispositif de mesure selon l'une des revendications précédentes, en ce que le capteur de flexion (3, 4, 15) est un capteur de flexion multimorphe composé de plusieurs paires de capteurs ayant une polarité antiparallèle alternée.
